(19) Europäisches Patentamt / European Patent Office / Office européen des brevets

(11) **EP 4 779 013 A1**

(12) **EUROPEAN PATENT APPLICATION**
published in accordance with Art. 153(4) EPC

(43) Date of publication:
**22.07.2026 Bulletin 2026/30**

(21) Application number: 25788008.8

(22) Date of filing: **13.05.2025**

(51) International Patent Classification (IPC):
$C12N\ 1/20^{(2026.01)}$   $A61K\ 35/745^{(2015.01)}$
$A61K\ 35/744^{(2015.01)}$   $A61P\ 1/16^{(2006.01)}$
$A61P\ 39/06^{(2006.01)}$   $C12N\ 1/04^{(2006.01)}$
$C12R\ 1/01^{(2006.01)}$   $C12R\ 1/225^{(2006.01)}$

(86) International application number:
**PCT/CN2025/094610**

(87) International publication number:
**WO 2026/040498 (26.02.2026 Gazette 2026/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **19.08.2024 CN 202411132472**

(71) Applicant: **Wecare Probiotics Co., Ltd.**
**Suzhou, Jiangsu 215200 (CN)**

(72) Inventors:
• FANG, Shuguang
  Suzhou, Jiangsu 215200 (CN)
• QI, Yongmei
  Suzhou, Jiangsu 215200 (CN)
• DONG, Yao
  Suzhou, Jiangsu 215200 (CN)
• GAI, Zhonghui
  Suzhou, Jiangsu 215200 (CN)
• ZHU, Jianguo
  Suzhou, Jiangsu 215200 (CN)

(74) Representative: **Bauer, Karel**
**Uresova 1266/2**
**148 00 Praha 4 (CZ)**

(54) **COMPOSITE PROBIOTIC FOR RELIEVING OXIDATIVE-STRESS-INDUCED LIVER DAMAGE, AND USE THEREOF**

(57) The present invention involves a compound probiotic for alleviating oxidative stress-induced liver damage and use thereof, and the compound probiotic for alleviating oxidative stress-induced liver damage consists of the *Bifidobacterium animalis subsp. lactis* BLa36 strain and the *Lactobacillus paracasei* LC86 strain. It is founded in the present invention that the two strains mentioned above can cooperate with each other for mutual promotion, thus synergizing the effect of preventing and treating oxidative stress-induced liver damage. This is specifically manifested in the following aspects: effectively reducing the malondialdehyde (MDA) content in the liver of a rat model with D-galactose-induced acute liver damage, increasing the activities of total superoxide dismutase, catalase and glutathione peroxidase in the liver, and enhancing its antioxidant capacity; effectively reducing the TNF-α content in the liver of a rat model with D-galactose-induced acute liver damage, and enhancing the immune level of the body.

Fig. 1

Processed by Luminess, 75001 PARIS (FR)

## Description

## Technical Field

The present invention belongs to the technical field of probiotics, and relates to a compound probiotic for alleviating oxidative stress-induced liver damage and use thereof.

## Background Art

Oxidative stress, a physiological response of the body under various internal and external environmental stimuli, may lead to the generation of a large number of oxygen free radicals with high oxidation ability, which can damage liver cells and thereby cause abnormal liver function. Oxidative stress-induced liver damage is one of the important factors for onset of liver diseases.

For oxidative stress-induced liver damage, traditional treatment methods mainly include medication and lifestyle intervention. However, medication is often accompanied by some side effects, and its long-term efficacy is not stable. Lifestyle intervention, though important, is difficult to adhere to in practice, and moreover, its effect varies from person to person.

In recent years, the role of probiotics in regulating the balance of intestinal microecology and enhancing the body's immunity has been gradually discovered and valued. Especially in the adjutant therapies for liver diseases, probiotics have shown remarkable effects. Specific types of probiotics can reduce the oxidative stress level of the liver through multiple pathways, e.g., improving liver detoxification function, restoring liver microecological balance, and enhancing liver immune function. These findings have provided ideas for the development of new treatment methods for liver damage.

There are a wide variety of probiotic products on the current market, but specialized products for oxidative stress-induced liver damage are not common. In addition, different probiotic strains have different functions or effects, and so, how to select and combine these strains to achieve the best therapeutic effect is also an urgent problem to be solved.

## Contents of the Invention

In view of the deficiencies of the prior art, the present invention aims to provide a compound probiotic for alleviating oxidative stress-induced liver damage and use thereof.

To attain the above object, the present invention employs the following technical solution:

In a first aspect, the present invention provides a compound probiotic for alleviating oxidative stress-induced liver damage, which consists of *Bifidobacterium animalis subsp. lactis* BLa36 strain with a collection number of CGMCC No. 24029 and *Lactobacillus paracasei* LC86 strain with a collection number of CGMCC No.1.12731.

For the present invention, a brand-new probiotic compounding method and a brand-new strategy for the prevention and treatment of oxidative stress-induced liver damage have been creatively developed, that is, after the *Bifidobacterium animalis subsp. lactis* BLa36 strain and the *Lactobacillus paracasei* LC86 strain are compounded for combination use, it is found that the two can cooperate with each other for mutual promotion, thus synergizing the effect of preventing and treating oxidative stress-induced liver damage. This is specifically manifested in the following aspects: (1) effectively reducing the malondialdehyde (MDA) content in the liver of a rat model with D-galactose-induced acute liver damage, increasing the activities of total superoxide dismutase, catalase and glutathione peroxidase in the liver, and enhancing its antioxidant capacity; (2) effectively reducing the TNF-$\alpha$ content in the liver of a rat model with D-galactose-induced acute liver damage, and enhancing the immune level of the body. Compared with the intervention method with only one strain, the compounding of the two strains is significantly more effective in alleviating oxidative stress-induced liver damage under the same dosage. At the same time, both stains are probiotics, and so the product is safer and resistance to the product will not be easily generated.

Preferably, the viable count ratio of the BLa36 strain to the LC86 strain is in the ranged of 1:5 to 5:1, e.g., 1:5, 1:4, 1:3, 1:2, 1:1, 2:1, 3:1, 4:1, 5:1, etc. Other specific values in the above numerical range can also be selected, which will not be detailed here.

In the second aspect, the present invention provides a probiotic preparation for alleviating oxidative stress-induced liver damage, and the strain in the probiotic preparation is the compound probiotic described in the first aspect.

Preferably, the viable content of the compound probiotic in the probiotic preparation is not less than $1 \times 10^9$ CFU/g or $1 \times 10^9$ CFU/mL, for example, the viable content is $1 \times 10^9$ CFU/g (CFU/mL), $1 \times 10^{10}$ CFU/g (CFU/mL), $5 \times 10^{10}$ CFU/g (CFU/mL), $1 \times 10^{11}$ CFU/g (CFU/mL), $3 \times 10^{11}$ CFU/g (CFU/mL), $5 \times 10^{11}$ CFU/g (CFU/ mL), $1 \times 10^{12}$ CFU/g (CFU/mL), $1 \times 10^{13}$ CFU/g (CFU/mL), etc. Other specific values in this range can also be selected, which will not be detailed here.

Preferably, the dosage forms of the probiotic preparation include solution, freeze-dried powder, capsules, tablets or granules. The dosage forms of the probiotic preparation in this invention are not limited, that is, the dosage forms can include the most commonly used solution, freeze-dried powder, further-prepared capsules, tablets or granules.

Preferably, solution is adopted as the dosage form of the probiotic preparation, which is prepared by the following method:

Inoculate the BLa36 strain and LC86 strain in the media respectively for activation and fermentation culture, so as to obtain the fermentation broths. Centrifuge the fermentation broths respectively, and then resuspend with solvent to obtain BLa36 bacterial suspension and LC86 bacterial suspension. Mix BLa36 bacterial suspension and LC86 bacterial suspension based on the viable count ratio to obtain the probiotic preparation.

Preferably, freeze-dried powder is adopted as the dosage form of the probiotic preparation, which is prepared by the following method:

Inoculate the BLa36 strain and LC86 strain in the media respectively for activation and fermentation culture, so as to obtain the fermentation broths. Centrifuge the fermentation broths respectively, then mix with a protective agent and freeze-dry to obtain BLa36 bacterial powder and LC86 bacterial powder. Mix BLa36 bacterial powder and LC86 bacterial powder based on the viable count ratio to obtain the probiotic preparation.

In a third aspect, the present invention provides the use of compound probiotic described in the first aspect or probiotic preparation described in the second aspect in preparation of drugs for the prevention, alleviation or treatment of oxidative stress-induced liver damage.

Preferably, the oxidative stress-induced liver damage includes acute liver damage, nonalcoholic fatty liver disease, alcoholic liver disease, or viral hepatitis.

Preferably, the drugs also contain excipients.

The excipients include any one or at least two of fillers, binders, wetting agents, disintegrants, emulsifiers, co-solvents, solubilizers, osmotic pressure regulators, colorants, pH adjusters, antioxidants, bacteriostatic agents or buffers.

Compared with the prior art, the present application has the following beneficial effects:

For the present invention, a brand-new probiotic compounding method and a brand-new strategy for the prevention and treatment of oxidative stress-induced liver damage have been creatively developed, that is, after the *Bifidobacterium animalis subsp. lactis* BLa36 strain and the *Lactobacillus paracasei* LC86 strain are compounded for combination use, it is found that the two can cooperate with each other for mutual promotion, thus synergizing the effect of preventing and treating oxidative stress-induced liver damage. This is specifically manifested in the following aspects: (1) effectively reducing the malondialdehyde (MDA) content in the liver of a rat model with D-galactose-induced acute liver damage, increasing the activities of total superoxide dismutase, catalase and glutathione peroxidase in the liver, and enhancing its antioxidant capacity; (2) effectively reducing the TNF-$\alpha$ content in the liver of a rat model with D-galactose-induced acute liver damage, and enhancing the immune level of the body. Compared with the intervention method with only one strain, the compounding of the two strains is significantly more effective in alleviating oxidative stress-induced liver damage under the same dosage. At the same time, both stains are probiotics, and so the product is safer and resistance to the product will not be easily generated.

According to the Classified Nomenclature, the BLa36 strain involved in the present invention is named as *Bifidobacterium animalis subsp. Lactis,* which was collected by the China General Microbiological Culture Collection Center on December 2, 2021, with a collection number of CGMCC No.24029, at the following address: No. 3, Yard 1, Beichen West Road, Chaoyang District, Beijing.

According to the Classified Nomenclature, the LC86 strain involved in the present invention is named as *Lactobacillus paracasei,* which was collected by the China General Microbiological Culture Collection Center on July 20, 2020, with a collection number of CGMCC No.1.12731, at the following address: No. 3, Yard 1, Beichen West Road, Chaoyang District, Beijing.

**Description of Drawings**

Fig. 1 shows the statistical results of malondialdehyde (MDA) level in the liver of each group of rats.

Fig. 2 shows the statistical results of total superoxide dismutase (T-SOD) activity in the liver of each group of rats.

Fig. 3 shows the statistical results of catalase (CAT) activity in the liver of each group of rats.

Fig. 4 shows the statistical results of glutathione peroxidase (GSH-Px) activity in the liver of each group of rats.

Fig. 5 shows the statistical results of tumor necrosis factor-$\alpha$ (TNF-$\alpha$) level in the liver of each group of rats.

**Embodiments**

The technical solution of the present invention will be further detailed below in embodiments. Those skilled in the art should appreciate that the examples described herein are only intended to assist in understanding of the present invention, and should not be regarded as specific limitation on the present application.

The formulation of the medium involved in the following examples is as following:

MRS medium: peptone 10g/L, beef extract 10g/L, glucose 20g/L, sodium acetate 2g/L, yeast powder 5g/L, diammonium hydrogen citrate 2g/L, $K_2PO_4 \cdot 3H_2O$ 2.6g/L, $MgSO_4 \cdot 7H_2O$ 0.1g/L, $MnSO_4$ 0.05g/L, Tween 80 1mL/L, cysteine hydrochloride 0.5g/L.

According to the Classified Nomenclature, the BLa36 strain involved in the following examples is named as *Bifidobacterium animalis subsp. lactis,* which was collected on December 2, 2021, under the collection No. of CGMCC No.24029.

According to the Classified Nomenclature, the LC86 strain involved in the following examples is named as *Lactobacillus paracasei,* which was collected on July 20, 2020, under the collection No. of CGMCC No.1.12731.

Preparation method for the bacterial suspension mentioned below: take out the required strain from the -80 °C freezer, inoculate it in liquid MRS medium at a dose of 2% (v/v), and incubate at 37 °C for 24 hours for activation, obtain activate fluid after 2 times of continuous activation; inoculate the activate fluid in liquid MRS medium at a dose of 2% (v/v), and incubate at 37 °C for 24 h to obtain a bacterial suspension; centrifuge the bacterial suspension at 5,000 rpm at 4 °C for 10min, filter to obtain the bacterial cells, and then resuspend with PBS solution to obtain the bacterial suspension.

The test results are statistically analyzed using ggplot2 in R language. For a companion with the control group, ### represents p<0.001, ## represents p<0.01, # represents p<0.05, and for a companion with the model group, *** represents p<0.001, ** represents p<0.01, * represents p<0.05, and NS. represents no significant difference.

Example 1

Evaluation of free radical scavenging activity:

(1) Incubate BLa36 strain and LC86 strain in liquid MRS medium at 37°C for 24 hours respectively, then centrifuge (12,000 g, 5 min, 4 °C) to collect the bacterial cells, wash the sediment of bacterial cells with PBS twice, resuspend the bacterial cells, and then adjust the cell concentration to $10^9$ CFU/mL. Add 1mL of bacterial suspension to 2 mL of 0.2 mmol/L DPPH absolute ethanol solution, mix well, and place the mixture at room temperature away from light for 30 min of reaction. Centrifuge at 10,000 rpm for 2 min, take the supernatant to measure its absorbance at 517 nm wavelength, and calculate the scavenging rate of probiotic strain for DPPH free radicals with the following formula:

$$\text{DPPH scavenging rate (\%)} = [1 - (\text{A sample group} - \text{A blank group})/\text{A control group}] \times 100$$

The difference between the blank group and the sample group is that the same volume of absolute ethanol was used to replace the DPPH solution, and the difference between the control group and the sample group is that the same volume of distilled water is used to replace the DPPH solution.

As shown in Table 1, both the BLa36 strain and LC86 strain involved in the presenbt invention had excellent DPPH free radical scavenging effect.

Table 1

| Strain | DPPH free radical scavenging rate |
|--------|-----------------------------------|
| BLa36  | 88.18 %                           |
| LC86   | 80.37 %                           |

(2) Incubate BLa36 strain and LC86 strain in liquid MRS medium at 37°C for 18 hours respectively, then centrifuge (12,000 g, 5 min, 4 °C) to collect the sediment, then resuspend the sediment in phosphate buffer saline (PBS), and adjust the viable count to $10^8$ CFU/mL, so as to obtain intact viable cell (IC). After ultrasonication of IC, centrifuge (8,000 g, 10 min, 4 °C) to remove the sediment, and collect the supernatant as cell-free extract (CFE).

(2.1) Determination of hydroxyl radical scavenging activity: place 1 mL of phenanthroline (0.1%, w/v), 1 mL of PBS (pH 7.4), 1 mL of 2.5 mM $FeSO_4$, 1 mL of 20 mM $H_2O_2$, and 0.5 mL of cell-free extract (CFE) or 0.5 mL of viable bacterial suspension (IC) ($1 \times 10^8$ CFU/mL) in a test tube, which is then placed in a water bath at 37°C for 1.5 hours of incubation. Measure the absorbance at 536 nm and calculate the hydroxyl radical scavenging rate with the following formula:

$$\text{Scavenging rate (\%)} = [(\text{A sample-A0})/(\text{A1-A0})] \times 100$$

Wherein A sample represents the absorbance value after CFE or IC treatment, A0 represents the absorbance value when 1 mL of $H_2O_2$ is replaced by 1 mL of distilled water, and A1 represents the absorbance value when 0.5 mL of sample is replaced by 0.5 mL of distilled water.

(2.2) Determination of superoxide anion scavenging activity: mix Tris-HCl buffer (0.05 M, pH 8.2, 2.8 mL), pyrogallol (0.05 M, 0.1 mL) and sample (CFE or IC) (0.1 mL) well, and incubate at 25°C away from light for 4 minutes. Add HCl (8 M, 1 mL) to stop the reaction. Measure the absorbance at 320 nm and calculate the superoxide anion scavenging rate with the following formula:

$$\text{Scavenging rate (\%)} = [1-(\text{A sample/A blank})] \times 100$$

Wherein A sample represents the absorbance value after CFE or IC treatment, and A blank represents the absorbance value when 0.1 mL of sample is replaced by 0.1 mL of distilled water.

As shown in Table 2, the BLa36 strain and LC86 strain involved in the present invention had excellent hydroxyl radical and superoxide anion scavenging effects.

Table 2

| Strain | Hydroxyl radical scavenging rate (%) | | Superoxide anion scavenging rate (%) | |
|---|---|---|---|---|
| | IC | CFE | IC | CFE |
| BLa36 | 61.46± 1.18 | 57.19 ± 1.51 | 47.24 ± 1.36 | 50.36 ± L38 |
| LC86 | 53.19± 1.05 | 55.92 ±0.98 | 43.82 ± 1.41 | 45.17± 1.12 |

Example 2

Improvement effect on the rat model with acute liver damage:

(1) Test animals: Healthy 8-week-old male Sprague Dawley rats (64 rats). These rats were raised in a controlled environment under a 12h light/dark cycle, room temperature: 21-23°, humidity: 50-60%. The animals could eat and drink freely.

(2) Animal grouping: after 1 week of adaptive feeding, the rats were randomly divided into 8 groups with 8 rats in each group: control group (Con), model group (D-gal), BLa36 group (intervention with BLa36 bacterial suspension), LC86 group (intervention with LC86 bacterial suspension), BLa36+LC86 group 1 (combined intervention with BLa36 bacterial suspension and LC86 bacterial suspension, viable count ratio: 1:1), BLa36+ LC86 group 2 (combined intervention with BLa36 bacterial suspension and LC86 bacterial suspension, viable count ratio: 5:1), BLa36+LC86 group 3 (combined intervention with BLa36 bacterial suspension and LC86 bacterial suspension, viable count ratio: 1:5), ATCC700541+LC86 group (combined intervention with commercially available Bifidobacterium animalis subsp. lactis ATCC700541 bacterial suspension and LC86 bacterial suspension, viable count ratio: 1:1).

(3) Animal modeling and intervention methods:

Control group (Con): normal saline was administered intragastrically every day (20 ml/kg body weight);

Model group (D-gal): normal saline was administered intragastrically every day (20 ml/kg body weight) and 5% (w/v) D-galactose dissolved in normal saline was injected subcutaneously (500 mg/kg body weight) once every day, duration: 8 weeks;

Probiotic intervention groups: probiotic solutions were administered intragastrically every day (total dosage: $10^9$ CFU/day/kg body weight), and 5% (w/v) D-galactose dissolved in normal saline was injected subcutaneously (500 mg/kg body weight) once every day, duration: 8 weeks.

(4) Analysis of indicators:

(4.1) Analysis of oxidative stress markers in rat liver:

After the test results were obtained, the rats were sacrificed, the rat livers were removed and rinsed with nuclease-free water, and then, the liver was used for preparing 10% (w/v) homogenate by adding a sterile 0.9% saline solution. The supernatant was taken for kit detection of total superoxide dismutase (T-SOD), catalase (CAT), glutathione peroxidase (GSH-Px) and malondialdehyde (MDA) which are antioxidant indicators of the liver, so as to evaluate the antioxidant capacity of the rat liver and the degree of rat liver damage.

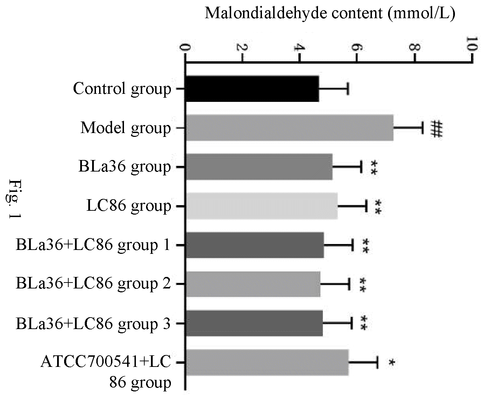

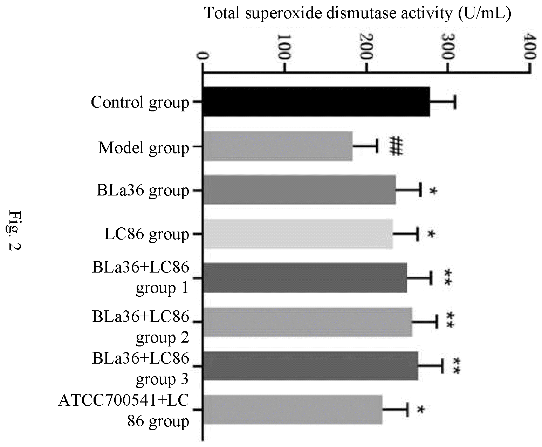

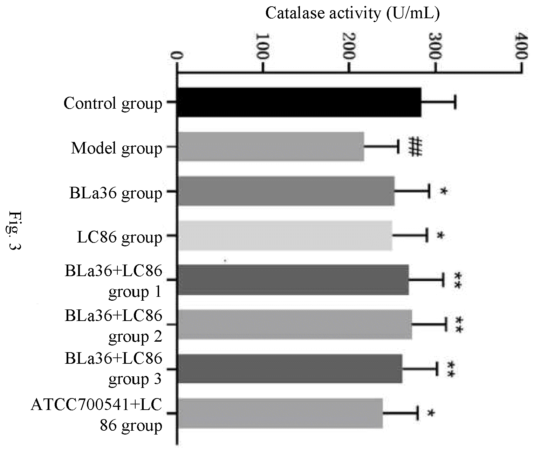

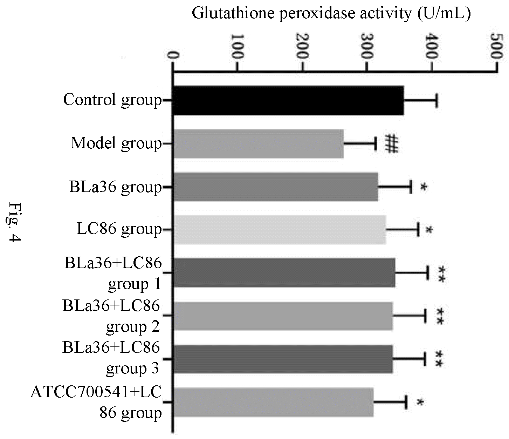

As shown in Figs. 1-4, compared with the control group, in the model group, the level of malondialdehyde (MDA) was significantly increased, and the activities of total superoxide dismutase (T-SOD), catalase (CAT), and glutathione peroxidase (GSH-Px) were significantly decreased. After the intervention of various probiotics, the malondialdehyde (MDA) levels in the livers of rats were decreased to varying degrees, and the activities of total superoxide dismutase (T-SOD), catalase (CAT) and glutathione peroxidase (GSH-Px) increased to varying degrees. In particular, the effects of combined intervention of BLa36 and LC86 were more prominent.

(4.2) Analysis of inflammatory factors of rat livers:

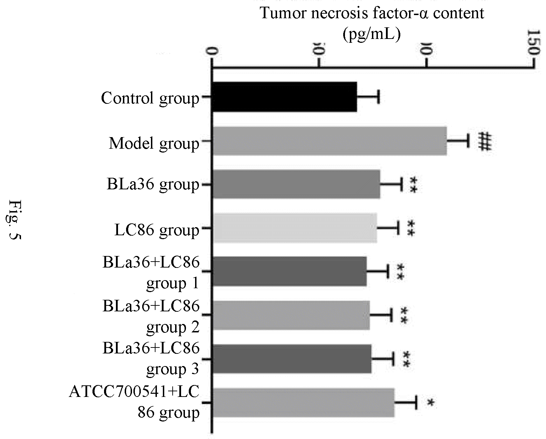

After the test results were obtained, the rats were sacrificed, the rat livers were removed and rinsed with nuclease-free water, and then, the liver was used for preparing 10% (w/v) homogenate by adding a sterile 0.9% saline solution. The supernatant was taken for kit detection of the content of tumor necrosis factor-$\alpha$ (TNF-$\alpha$) in the rat livers.
As shown in Fig. 5, compared with the control group, the level of tumor necrosis factor -$\alpha$ (TNF-$\alpha$) in the model group was significantly increased. After the intervention of various probiotics, the tumor necrosis factor-$\alpha$ (TNF-$\alpha$) levels in the livers of rats were decreased to varying degrees. In particular, the effect of combined intervention of BLa36 and LC86 was more prominent, and the effect is more closer to that of the control group.
The applicant states: the technical solution of the present invention is illustrated through the above examples, but the present application is not limited to those examples, which is to say, the present application can be implemented without relying on the above-mentioned examples. Those skilled in the art should appreciate that any improvement made to the present application, equivalent replacement of raw materials and addition of auxiliary components in the product of the present invention , or selection of specific methods, etc. fall within the scope of protection and disclosure of the present application.
The above provides a detailed description of the preferred embodiment of the present invention, however, the present invention is not limited to the specific details mentioned in the above-mentioned embodiments. Those skilled in the art can make several simple modifications to the technical solution of the present invention, without departing from the technical spirit of the present invention. However, all those modifications shall be deemed as falling into the protection scope of the present invention.
In addition, it should be noted that the specific technical features described in above embodiments can be combined in any appropriate form, provided that there is no conflict. In order to avoid unnecessary repetition, the present invention will not elaborate on all possible combinations any further.

**Claims**

1.  A compound probiotic for alleviating oxidative stress-induced liver damage, **characterized in that** the compound probiotic for alleviating oxidative stress-induced liver damage consists of *Bifidobacterium animalis subsp. lactis* BLa36 strain with a collection No. of CGMCC No.24029 and *Lactobacillus paracasei* LC86 strain with a collection No. of CGMCC No.1.12731.

2.  The compound probiotic for alleviating oxidative stress-induced liver damage according to claim 1, **characterized in that** the viable count ratio of the BLa36 strain to the LC86 strain is in the range of 1:5 to 5:1.

3.  A probiotic preparation for alleviating oxidative stress-induced liver damage, **characterized in that** the strain contained in the probiotic preparation is the compound probiotic according to claim 1 or 2.

4.  The probiotic preparation according to claim 3, **characterized in that** the viable content of the compound probiotic in the probiotic preparation is not less than $1\times10^9$ CFU/g or $1\times10^9$ CFU/mL.

5.  The probiotic preparation according to claim 3, **characterized in that** the dosage forms of the probiotic preparation include solution, freeze-dried powder, capsules, tablets or granules.

6.  The probiotic preparation according to claim 5, **characterized in that** solution is adopted as the dosage form of the probiotic preparation, which is prepared by the following method:
    inoculate the BLa36 strain and LC86 strain in the media respectively for activation and fermentation culture, so as to obtain fermentation broths; centrifuge the fermentation broths respectively, and then resuspend with solvent to obtain BLa36 bacterial suspension and LC86 bacterial suspension; mix BLa36 bacterial suspension and LC86 bacterial suspension based on the viable count ratio to obtain the probiotic preparation.

7. The probiotic preparation according to claim 5, **characterized in that** freeze-dried powder is adopted as the dosage form of the probiotic preparation, which is prepared by the following method:
inoculate the BLa36 strain and LC86 strain in the media respectively for activation and fermentation culture, so as to obtain fermentation broths; centrifuge the fermentation broths respectively, then mix with a protective agent and freeze-dry to obtain BLa36 bacterial powder and LC86 bacterial powder; mix BLa36 bacterial powder and LC86 bacterial powder based on the viable count ratio to obtain the probiotic preparation.

8. Use of compound probiotic according to claim 1 or 2 or probiotic preparation according to any one of claims 3 to 7 in preparation of drugs for the prevention, alleviation or treatment of oxidative stress-induced liver damage.

9. The use according to claim 8, **characterized in that** the oxidative stress-induced liver damage mentioned above includes acute liver damage, non-alcoholic fatty liver disease, alcoholic liver disease or viral hepatitis.

10. The use according to claim 8, **characterized in that** the drugs also contain excipients;
the excipients include any one or at least two of fillers, binders, wetting agents, disintegrants, emulsifiers, co-solvents, solubilizers, osmotic pressure regulators, colorants, pH adjusters, antioxidants, bacteriostatic agents or buffers.

Fig. 1

Fig. 2

Fig. 3

Fig. 4

Fig. 5

## INTERNATIONAL SEARCH REPORT

International application No.

**PCT/CN2025/094610**

| A. | CLASSIFICATION OF SUBJECT MATTER |
|---|---|

C12N 1/20(2006.01)i;  A61K 35/745(2015.01)i;  A61K 35/744(2015.01)i;  A61P 1/16(2006.01)i;  A61P 39/06(2006.01)i;  C12N 1/04(2006.01)i;  C12R 1/01(2006.01)i;  C12R 1/225(2006.01)i

According to International Patent Classification (IPC) or to both national classification and IPC

| B. | FIELDS SEARCHED |
|---|---|

Minimum documentation searched (classification system followed by classification symbols)

IPC:C12N,A61K,A61P,C12R

Documentation searched other than minimum documentation to the extent that such documents are included in the fields searched

Electronic data base consulted during the international search (name of data base and, where practicable, search terms used)

CNTXT, VEN, VCN, ENTXTC, OETXT, CNKI, PubMed, ISI-WEB OF SCIENCE: 肝损, Liver, injury, 氧化应激, oxidative stress, 动物双歧杆菌乳亚种, 乳双歧杆菌, Bifidobacterium animalis subsp.lactis, Bifidobacterium lactis, BLa36, CGMCC No.24029, 副干酪乳杆菌, Lactobacillus paracasei, LC86, CGMCC No.1.12731, ATCC PTA-126816, DSM 35077

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 118638700 A (WECARE PROBIOTICS (SUZHOU) CO., LTD.) 13 September 2024 (2024-09-13)<br>    see abstract, and claims 1-10 | 1-10 |
| Y | CN 117987327 A (WECARE PROBIOTICS (SUZHOU) CO., LTD.) 07 May 2024 (2024-05-07)<br>    see abstract, and claims 1-10 | 1-10 |
| Y | CN 116617273 A (WECARE PROBIOTICS (SUZHOU) CO., LTD.) 22 August 2023 (2023-08-22)<br>    see abstract, and claims 1-10 | 1-10 |
| A | CN 114350577 A (WECARE PROBIOTICS (SUZHOU) CO., LTD.) 15 April 2022 (2022-04-15)<br>    see abstract, and claims 1-10 | 1-10 |
| A | CN 115466696 A (THANKCOME BIOLOGICAL SCIENCE AND TECHNOLOGY (SUZHOU) CO., LTD) 13 December 2022 (2022-12-13)<br>    see abstract, and claims 1-8 | 1-10 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 August 2025** | **10 September 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2025/094610** |

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 117286077 A (WECARE PROBIOTICS (SUZHOU) CO., LTD.; LUOHE WECARE BIOTECHNOLOGY CO., LTD.) 26 December 2023 (2023-12-26)<br>see abstract, and claims 1-10 | 1-10 |
| A | CN 114145460 A (LUNAN PHARMACEUTICAL GROUP CORP.) 08 March 2022 (2022-03-08)<br>see abstract, and claims 1-20 | 1-10 |

Form PCT/ISA/210 (second sheet) (July 2022)

**INTERNATIONAL SEARCH REPORT**
Information on patent family members

International application No.

**PCT/CN2025/094610**

| Patent document cited in search report | | | Publication date (day/month/year) | Patent family member(s) | Publication date (day/month/year) |
|---|---|---|---|---|---|
| CN | 118638700 | A | 13 September 2024 | None | |
| CN | 117987327 | A | 07 May 2024 | None | |
| CN | 116617273 | A | 22 August 2023 | None | |
| CN | 114350577 | A | 15 April 2022 | None | |
| CN | 115466696 | A | 13 December 2022 | None | |
| CN | 117286077 | A | 26 December 2023 | None | |
| CN | 114145460 | A | 08 March 2022 | None | |

Form PCT/ISA/210 (patent family annex) (July 2022)